# FASCICULE DE BREVET EUROPEEN

(11) **EP 0 497 929 B1**
(45) Date de publication et mention de la délivrance du brevet: **13.03.1996**
(21) Numéro de dépôt: 91912152.5
(22) Date de dépôt: 20.06.1991
(51) Int. Cl.: A61L 2/04, A61K 35/16

(54) **COMPOSITION POUR STABILISER LE PLASMA SANGUIN EN COURS DE PASTEURISATION**
ZUSAMMENSETZUNG ZUR STABILISIERUNG VON BLUTPLASMA IM LAUFE DER PASTEURISATION
COMPOSITION FOR STABILIZING BLOOD PLASMA DURING PASTEURIZATION

(30) Priorité: 03.07.1990 FR 9008375
(43) Date de publication de la demande: 12.08.1992
(73) Titulaire: CENTRE REGIONAL DE TRANSFUSION SANGUINE DE LILLE, F-59012 Lille (FR)
(72) Inventeur: BURNOUF, Myriana, F-59136 Wavrin (FR); BURNOUF, Thierry, F-59136 Wavrin (FR)
(74) Mandataire: Lhuillier, René
(86) Numéro de dépôt international: FR9100493
(87) Numéro de publication internationale: WO9200767

(56) Documents cités:
- EP-A- 0 053 338
- EP-A- 0 137 428
- EP-A- 0 176 926
- WO-A-89/06547
- Chemical Abstracts, vol. 101, no. 14, 1 octobre 1984, Columbus, Ohio, US; A.J. MACLEOD et al.: "Stabilization of proteins to heat", voir page 345, résumé 116629v

## Description

L'invention concerne une composition permettant de stabiliser le plasma sanguin en cours de pasteurisation, un procédé utilisant ladite composition et les solutions plasmatiques ainsi obtenues, particulièrement destinées aux thérapies de substitution de plasma et des facteurs V, XI et XIII de la coagulation sanguine.

Le plasma humain total ou dépourvu de cryoprotéines est encore utilisé comme thérapie de substitution pour les grands brûlés, les sujets gravement traumatisés ou ayant subi d'importantes interventions chirurgicales, c'est-à-dire dans tous les cas où les patients subissent d'importantes pertes de fluides.

On utilise généralement, pour ce type de thérapie, du plasma provenant de dons unitaires, de sujets sains et préalablement contrôlés, pour éviter les risques de transmission de maladies virales. Cette procédure ne permet cependant pas d'éliminer tous les risques de contamination par des virus en phase pré-sérologique, en particulier les différents virus de l'hépatite et le virus du SIDA.

Il serait donc avantageux de mettre au point une méthode d'inactivation virale qui n'altère pas les différentes fonctions biologiques du plasma.

Il a été bien démontré que le virus de l'Hépatite B était totalement inactivé par un chauffage à 60°C pendant 10 heures, en présence de citrate de sodium 0,5 M (Tabor et al. Thrombosis Res. 22, 1981, 233-238).Toutefois ce traitement entraîne une perte d'activité biologique de certaines protéines du plasma (Tabor et al. et Barrowcliffe et al. Fr. J. Haematology 55, 1983, 37-46).

Différentes protéines d'intérêt thérapeutique, purifiées à partir du plasma sanguin, ont pu être soumises à une pasteurisation classique à 60°C pendant 10 heures en présence de divers stabilisants. On constate toutefois que des molécules qui stabilisent une activité biologique peuvent être totalement inefficaces sur une autre.

Ainsi l'albumine peut être stabilisée par l'acétyl-tryptophane et le caprylate (Gellis et al. J. Clin. Invest. 27, 1948, 239-244) alors que ces stabilisants sont sans effet sur le plasminogène. La thrombine peut être stabilisée par des concentrations élevées de glycosides (Seegers.Arch. Biochem. 3, 1944, 363-367) alors que celles-ci ne protègent pas la prothrombine. L'addition d'un acide aminé et d'un glucide a donné de bons résultats avec le Facteur VIII et l'antithrombine III (voir brevet DE 29.16.711).

Le brevet européen 0 035 204 met en évidence une stabilisation de l' α antitrypsine, de l'antithrombine III, de la prékallikréine, de la fibronectine et du Facteur VIII en présence d'un polyol, celui-ci n'étant exemplifié que par le saccharose ; il signale cependant que dans les mêmes conditions le Facteur IX et la prékallikréine perdent toute leur activité thérapeutique.

Ces différentes données expérimentales confirment l'idée généralement admise que l'inactivation virale du plasma total (plasma frais, plasma frais congelé ou surnageant de cryoprécipité) ne peut pas être réalisée par une pasteurisation.

Le besoin médical existant encore pour du plasma total, la Demanderesse a cherché à mettre au point une composition assurant la protection simultanée de l'activité biologique de tous les facteurs d'intérêt thérapeutique contre la dénaturation en cours de pasteurisation.

Ayant observé que le sorbitol offrait une certaine protection contre la dénaturation thermique, mais avec des résultats variables d'un échantillon de plasma à l'autre et avec de faibles rendements, la Demanderesse a cherché différents additifs dont le mélange avec le sorbitol augmenterait son pouvoir stabilisant.

Ainsi la composition selon l'invention est constituée d'un mélange de sorbitol, de chlorure de calcium, d'héparine et de lysine. Les concentrations des différents constituants sont ajustées pour donner une concentration finale dans le plasma à pasteuriser de
- 50 à 80 % en sorbitol et, de préférence, de 60 %
- 0,1 à 1 U/ml d'héparine et de préférence, de 0,5 U/ml
- 1 à 10 g/l de lysine et, de préférence , de 4 g/l
- 3 à 5 mM en CaCl₂ et de préférence, de 4 mM.

La composition selon l'invention est additionnée au plasma frais ou au plasma frais congelé-décongelé ou au plasma dépourvu des protéines du cryoprécipité, avant de soumettre celui-ci à une pasteurisation par chauffage à 60°C±1°C pendant 10 heures.

Après la pasteurisation, la température est abaissée progressivement jusqu'à 20°C et la solution est soumise à une dialyse pour éliminer le sorbitol et l'héparine. Le tampon de dialyse est à pH 7, contient du citrate de sodium à une concentration comprise entre 4 et 10 mM, du chlorure de calcium 4 mM, du chlorure de sodium 0.13 M et de la lysine à une concentration de 4 g/l. Des tampons de dialyse de composition différente peuvent aussi être utilisés en fonction des besoins. La solution est ensuite concentrée pour rétablir le dosage physiologique des protéines plasmatiques. La solution résultante est soumise à une filtration stérilisante, conditionnée puis congelée ou lyophilisée.

L'invention concerne également le procédé de pasteurisation du plasma qui comprend l'addition de la composition selon l'invention dans le plasma, avant son chauffage, et ensuite la dialyse du plasma pasteurisé contre le tampon tel que defini plus haut.

Ce procédé est particulièrement avantageux parce qu'il peut être appliqué à de grands lots de plasma provenant de plusieurs récoltes indépendantes. Plus particulièrement, la pasteurisation de lots de 100 à 200 litres ou plus permet, après avoir effectué les contrôles appropriés, de garantir une constance de qualité des lots.

D'autre part, les lots de plasma pasteurisés par le procédé selon l'invention peuvent également servir de produits de substitution pour les malades présentant des déficiences en facteurs de la coagulation dont il n'existe pas de concentrés purifiés tels que le Facteur V, le Facteur XI et le Facteur XIII.

L'invention concerne donc également les solutions plasmatiques pasteurisées obtenues par le procédé selon l'invention et plus particulièrement destinées - d'une part au traitement des déficiences en Facteurs V, XI et XIII de la coagulation et - d'autre part aux thérapies de remplacement nécessitant du plasma total ou du surnageant de cryoprécipité.

L'exemple suivant décrit un mode de réalisation de l'invention sans toutefois en limiter la portée.

### EXEMPLE :

Deux litres de plasma décongelé sont additionnés d'héparine (1000 U) de lysine (8 g) et de chlorure de calcium 220 mg. Ces deux produits sont ajoutés sous la forme de sels en poudre, le plasma étant soumis à une agitation douce. Puis, 1200 g de sorbitol non dissous sont versés progressivement.

La pasteurisation est réalisée dans un récipient de 5 litres par chauffage à 60°C pendant 10 heures à l'aide soit d'un bain-marie, soit dans une cuve thermostatée. Après pasteurisation, le sorbitol et l'héparine sont éliminés par dialyse avec un rein artificiel ou un système d'ultrafiltration tel que le système Pellicon Ⓜ sur cassettes, en utilisant un tampon citraté contenant de la lysine à une concentration de 4 g/l, du CaCl2 4mM et du NaCl 0,13 M. La dialyse peut être suivie d'une concentration sur le même matériel pour ramener les taux en facteurs de coagulation à près d'1 U/ml comme dans un plasma thérapeutique de bonne qualité. Le matériel est dialysé à une osmolarité de 370 mosmol/l et un pH de 7. Le produit est ensuite filtré stérilement, par exemple sur filtre Millipack Ⓜ 40 (Millipore) à pores de 0.22 µm. La répartition du matériel s'effectue dans des poches de plastique pour congélation ou dans des flacons pour lyophilisation.

La stabilisation du plasma lors de la pasteurisation et de l'ultrafiltration par addition de calcium, d'héparine et de lysine permet d'obtenir les rendements suivants, par rapport à (noté ci-dessous vs) un témoin ne contenant que de la lysine et du sorbitol.

| | |
|---|---|
| FVIIIc | 87 % vs 66 % |
| FVc | 77 % vs 35 % |
| FVIIc | 55 % vs 52 % |
| FIXc | 60 % vs 49 % |
| fibrinogène coagulable | 81 % vs 57 % |
| FXIII | 77 % vs 71 % |

Il n'est pas observé de signes d'activation des facteurs de coagulation en présence de cette quantité de calcium. Ainsi le rapport FVII bovin/FVII coagulant est de 0,95 vs 1,09 pour le témoin. La stabilité, testée en suivant l'activité de FVIII après 24 heures de conservation du produit à l'état liquide et à température ambiante, n'est pas altérée par rapport au témoin (récupération de 100 % de l'activité). Ceci est à l'appui d'un taux en PKA < 2 % pour les produits pasteurisés et témoins.

De plus des contrôles sur le rat, par injection intraveineuse de plasma ainsi pasteurisé, indiquent l'absence d'effet hypotenseur et ne révèlent aucune modification du rythme cardiaque.

## Revendications

1. Composition pour stabiliser le plasma sanguin en cours de pasteurisation caractérisée en ce qu'elle est constituée d'un mélange de sorbitol, d'héparine, de chlorure de calcium et de lysine.

2. Composition selon la revendication 1 caractérisée en ce que la concentration de sorbitol est comprise entre 500 et 800 g/l.

3. Composition selon la revendication 2 caractérisée en ce que la concentration de sorbitol est de 600 g/l.

4. Composition selon l'une quelconque des revendications 1 à 3 caractérisée en ce que la concentration d'héparine est comprise entre 100 et 1000 U/l.

5. Composition selon la revendication 4 caractérisée en ce que la concentration d'héparine est de 500 U/l.

6. Composition selon l'une quelconque des revendications 1 à 5 caractérisée en ce que la concentration en chlorure de calcium est comprise entre 3 et 5 mM.

7. Composition selon la revendication 6 caractérisée en ce que la concentration de chlorure de calcium est de 4 mM.

8. Composition selon l'une quelconque des revendications 1 à 7 caracterisée en ce que la concentration de lysine est comprise entre 1 et 10 g/l.

9. Composition selon la revendication 8 caractérisée en ce que la concentration de lysine est de 4 g/l.

10. Procédé de pasteurisation du plasma sanguin caractérisé en ce qu'il comprend l'addition de la composition selon l'une quelconque des revendications 1 à 9 dans le plasma, avant l'étape de chauffage, et, ensuite, l'élimination de ladite composition par dialyse.

11. Procedé selon la revendication 10 caractérisé en ce que la dialyse est effectuée à pH 7 contre du tampon contenant du citrate de sodium à une concentration comprise entre 4 et 10 mM, du chlorure de calcium 4 mM, du chlorure de sodium 0,13 M et de la lysine à une concentration de 4 g/l.

12. Procédé selon la revendication 10 ou 11 caractérisé en ce qu'il est applicable à des volumes de plasma compris entre 1 litre et plusieurs centaines de litres.

13. Solution plasmatique pasteurisée, à usage thérapeutique, destinée à compenser la déficience en Facteurs V, XI et XIII de la coagulation sanguine, caractérisée en ce qu'elle a été obtenue par le procédé selon l'une quelconque des revendications 10 à 12.

14. Plasma total pasteurisé, à usage thérapeutique, caractérisé en ce qu'il a été obtenu par le procédé selon l'une quelconque des revendications 10 à 12.

15. Surnageant de plasma cryoprécipité et dépourvu de cryoprotéines, pasteurisé, a usage thérapeutique, caractérisé en ce qu'il a été obtenu par le procédé selon l'une quelconque des revendications 10 à 12.

## Claims

1. Composition for stabilizing blood plasma during pasteurization, characterized in that it is formed of a mixture of sorbitol, heparin, calcium chloride and lysine.

2. Composition according to claim 1, characterized in that the sorbitol concentration is between 500 and 800 g/l.

3. Composition according to claim 2, characterized in that the sorbitol concentration is 600 g/l.

4. Composition according to any one of claims 1 to 3, characterized in that the heparin concentration is between 100 and 1000 U/l.

5. Composition according to claim 4,characterized in that the heparin concentration is 500 U/l.

6. Composition according to any one of claims 1 to 5, characterized in that the calcium chloride concentration is between 3 and 5 mM.

7. Composition according to claim 6, characterized in that the calcium chloride concentration is 4 mM.

8. Composition according to any one of claims 1 to 7, characterized in that the lysine concentration is between 1 and 10 g/l.

9. Composition according to claim 8, characterized in that the lysine concentration is 4 g/l.

10. A process for blood plasma pasteurization, characterized in that it includes the addition of the composition according to any one of claims 1 to 9 to the plasma, prior to the heating step, and, subsequently, the removal of the said composition by dialysis.

11. Process according to claim 10, characterized in that the dialysis is carried out at a pH of 7 against a buffer solution containing sodium citrate at a concentration of between 4 and 10 mM, 4 mM calcium chloride, 0.13 M sodium chloride and lysine in a concentration of 4 g/l.

12. Process according to claim 10 or 11, characterized in that it is applicable to volumes of plasma of between 1 litre and several hundreds of litres.

13. Plasma solution for therapeutic use intended to compensate for deficiency in blood coagulation factors V, XI and XIII, characterized in that it has been obtained using the process according to any one of claims 10 to 12.

14. Whole plasma for therapeutic use, characterized in that it has been pasteurized using the process according to any one of claims 10 to 12.

15. Supernatant of cryoprecipitated and cryoprotein-free plasma, for therapeutic use, characterized in that it has been pasteurized using the process according to any one of claims 10 to 12.

## Patentansprüche

1. Zusammensetzung zur Stabilisierung des Blutplasmas wahrend der Pasteurisierung, dadurch gekennzeichnet, daß sie aus einer Mischung von Sorbit, Heparin, Calciumchlorid und Lysin besteht.

2. Zusammensetzung nach Anspruch 1, dadurch gekennzeichnet, daß die Sorbitkonzentration zwischen 500 und 800 g/l liegt.

3. Zusammensetzung nach Anspruch 2, dadurch gekennzeichnet, daß die Sorbitkonzentration 600 g/l beträgt.

4. Zusammensetzung nach einem der Ansprüche 1 bis 3, dadurch gekennzeichnet, daß die Heparinkonzentration zwischen 100 und 1000 Einh./l liegt.

5. Zusammensetzung nach Anspruch 4, dadurch gekennzeichnet, daß die Heparinkonzentration 500 Einh./l beträgt.

6. Zusammensetzung nach einem der Ansprüche 1 bis 5, dadurch gekennzeichnet, daß die Calciumchloridkonzentration zwischen 3 und 5 mmol/l liegt.

7. Zusammensetzung nach Anspruch 6, dadurch gekennzeichnet, daß die Calciumchloridkonzentration 4 mmol/l beträgt.

8. Zusammensetzung nach einem der Ansprüche 1 bis 7, dadurch gekennzeichnet, daß die Lysinkonzentration zwischen 1 und 10 g/l liegt.

9. Zusammensetzung nach Anspruch 8, dadurch gekennzeichnet, daß daß die Lysinkonzentration 4 g/l beträgt.

10. Verfahren zur Blutplasma-Pasteurisierung, dadurch gekennzeichnet, daß es vor dem Schritt der Erwärmung die Zugabe der Zusammensetzung gemäß einem der Ansprüche 1 bis 9 zu dem Plasma und anschließend die Beseitigung der genannten Zusammensetzung durch Dialyse umfaßt.

11. Verfahren nach Anspruch 10, dadurch gekennzeichnet, daß die Dialyse bei pH 7 gegen einen Puffer erfolgt, der Natriumcitrat in einer Konzentration zwischen 4 und 10 mmol/l, 4 mmol/l Calciumchlorid, 0,13 mol/l Natriumchlorid und Lysin in einer Konzentration von 4 g/l enthält.

12. Verfahren nach Anspruch 10 oder 11, dadurch gekennzeichnet, daß es auf Plasmavolumina zwischen 1 Liter und mehreren Hundert Liter Anwendung findet.

13. Pasteurisierte Plasmalösung für den therapeutischen Gebrauch, bestimmt zur Kompensation des Mangels an den Blutgerinnungsfaktoren V, XI und XII, dadurch gekennzeichnet, daß sie durch das Verfahren nach einem der Ansprüche 10 bis 12 erhalten wurde.

14. Pasteurisiertes Gesamtplasma für den therapeutischen Gebrauch, dadurch gekennzeichnet, daß es durch das Verfahren nach einem der Ansprüche 10 bis 12 erhalten wurde.

15. Von Kryoproteinen freier, pasteurisierter Plasma-Kryopräzipitat-Überstand für den therapeutischen Gebrauch, dadurch gekennzeichnet, daß er durch das Verfahren nach einem der Ansprüche 10 bis 12 erhalten wurde.
